# EUROPEAN PATENT SPECIFICATION

(11) **EP 4 003 188 B1**
(45) Date of publication and mention of the grant of the patent: **27.08.2025**
(21) Application number: 20761928.9
(22) Date of filing: 30.07.2020
(51) Int. Cl.: A61B 17/08, A61B 17/10, A61B 17/122, A61B 17/128, A61B 90/00, A61B 17/02, A61B 17/00

(54) **SYSTEMS FOR ENDOSCOPIC SUBMUCOSAL DISSECTION**
SYSTEME FÜR ENDOSKOPISCHE SUBMUKOSALE DISSEKTION
SYSTÈMES DE DISSECTION SOUS-MUCOSALE ENDOSCOPIQUE

(30) Priority: 30.07.2019 US 201962880287 P
(43) Date of publication of application: 01.06.2022
(73) Proprietor: Covidien LP, Mansfield, MA 02048 (US)
(72) Inventor: CAMISA, William, Los Altos, CA 94024 (US); HUSZAR, Hillary K., Redwood City, CA 94065 (US)
(74) Representative: Maschio & Soames IP Ltd
(86) International application number: PCT/US2020/044157
(87) International publication number: WO 2021/021987

(56) References cited:
- US-A1- 2018 035 997
- US-A1- 2018 279 869
- US-A1- 2019 216 463

## Description

### FIELD

The present technology is related generally to tissue clips used in endoscopic submucosal dissection.

### BACKGROUND

Endoscopic resection has been accepted as a first choice of the treatment for early stage GI carcinomas because of less invasiveness and lower cost. Endoscopic submucosal resection (ESD) allows for an en bloc resection and accurate histopathological diagnosis regardless of the size, an existence of severe fibrosis at submucosal layer, and location of a lesion. One of the benefits of ESD is lower recurrence rates compared to endoscopic mucosal resection. During some ESD's, jaw members of a tissue clip are engaged to tissue. After performing the dissection, the jaw members are disengaged from the tissue.

US 2018/279869 discloses medical devices that lift and retract tissue during a dissection procedure to improve visualization of the target tissue and mitigate obstructions for dissection tools. In particular, such a device may transition from a constrained to an unconstrained bowed configuration to immobilize and retract the dissected portion of target tissue during a dissection procedure.

US 2019/216463 discloses an endoscopic surgical instrument icluding a hemostatic clip which is deployed during endoscopic submucosal resection. The hemostatic clip has an elongated collar configured to be detachably coupled to a shaft of the surgical instrument, a pair of jaws received in the collar, and a magnet coupled to the jaws.

US 2018/035997 discloses a tissue retraction system including a first anchor, a second anchor, and an elongate coupling member extending between the first anchor and the second anchor. The system also may include a holder for receiving the first anchor, the second anchor, and the elongate coupling element. The holder may include a proximal portion and a distal portion. The distal portion may have a smaller width than the proximal portion such that the distal portion exerts a force on a proximal end of the first anchor during deployment of the first anchor from the holder. The force may move the first anchor into an open configuration for receiving tissue.

### SUMMARY

The invention is defined in the appended claims

In one aspect, the present disclosure provides a tissue clip including a proximal body portion, first and second jaw members coupled to the proximal body portion, and a resilient member. The jaw members are configured to move between an open configuration and a closed configuration to grasp tissue therebetween. The resilient member is coupled to the proximal body portion or one of the first or second jaw members. The resilient member includes a segment that is laterally spaced from the proximal body portion when the resilient member is in a deployed state. The resilient member includes a first end portion coupled to the proximal body portion and extending distally therefrom, and a second end portion extending from the segment.

In aspects, the resilient member may be transitionable between a stored state and the deployed state.

In aspects, the segment of the resilient member may assume a linear shape when the resilient member is in the stored state, and the segment of the resilient member may assume an arcuate shape when the resilient member is in the deployed state.

In aspects, the segment of the resilient member may bow outwardly from the proximal body portion when the resilient member is in the deployed state.

In aspects, the segment may curve proximally and laterally from the first end portion.

In aspects, the second end portion may curve toward the proximal body portion.

In aspects, the second end portion may be disposed proximally of the first end portion.

In aspects, the resilient member may be configured to assume a looped configuration when in the deployed state.

In aspects, the resilient member may include a wire having a predefined shape.

In aspects, the resilient member may have a first end portion rotationally coupled to the proximal body portion, and a second end portion. The resilient member may be rotatable between a first position, in which the second end portion is disposed on a first side of the proximal body portion, and at least one second position, in which the second end portion is disposed on a second side of the proximal body portion.

In aspects, the proximal body portion may define an annular recess having the first end portion of the resilient member slidably received therein.

In aspects, the resilient member may include a wire wrapped about the proximal body portion.

In aspects, the first jaw member may define a pair of holes therethrough, and the resilient member may be attached to the first jaw member via the pair of holes.

In aspects, the resilient member may include first and second wires defining a gap therebetween.

In aspects, the gap may be greater than a diameter of the proximal body portion.

### BRIEF DESCRIPTION OF THE DRAWINGS

Objects and features of the presently disclosed tissue clips will become apparent to those of ordinary skill in the art when descriptions of various embodiments thereof are read with reference to the accompanying drawings, of which:
FIG. 1 is a top view illustrating an exemplary embodiment of a hand-held surgical instrument;
FIG. 2 is a side perspective view illustrating a tissue clip for operation by the surgical instrument of FIG. 1;
FIG. 3 is a top view of another embodiment of a tissue clip illustrated in a stored state within a sheath of the surgical instrument of FIG. 1;
FIG. 4 is a side perspective view of the tissue clip of FIG. 3 illustrated in a deployed state; and
FIG. 5 is a perspective view illustrating yet another embodiment of a surgical tissue clip shown without a resilient member.

### DETAILED DESCRIPTION

The present disclosure is generally directed to tissue clips including an elongate body, a pair of jaw members received in the elongate body and deployable therefrom, and a resilient member coupled to the jaw members or the elongate body. The resilient member is configured to assume a predefined, curved shape upon deployment of the tissue clip at a surgical site. During use, target tissue (e.g., a lesion) is grasped between the jaw members and the resilient member, in the deployed state, engages a gastric wall to separate the lesion from the gastric wall. With the resilient member maintaining the lesion in spaced relation from the gastric wall, the lesion may be dissected. In aspects, the resilient member may be swivelable about the elongate body to adjust a position thereof. These and other aspects of the present disclosure are described in greater detail below.

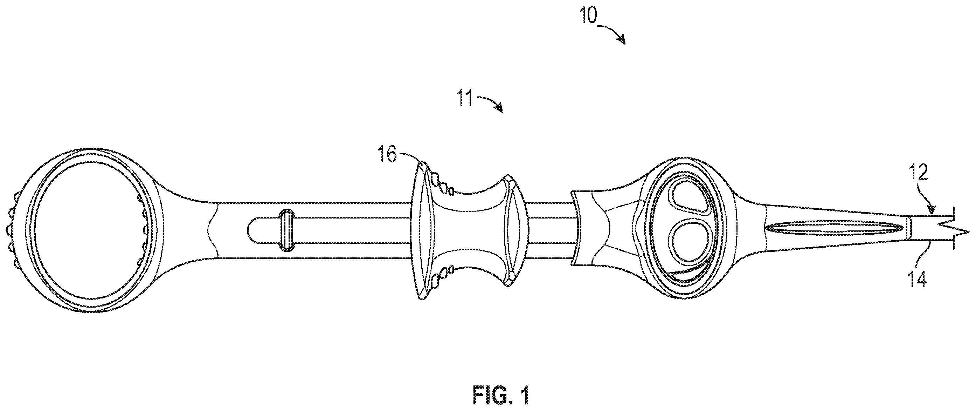

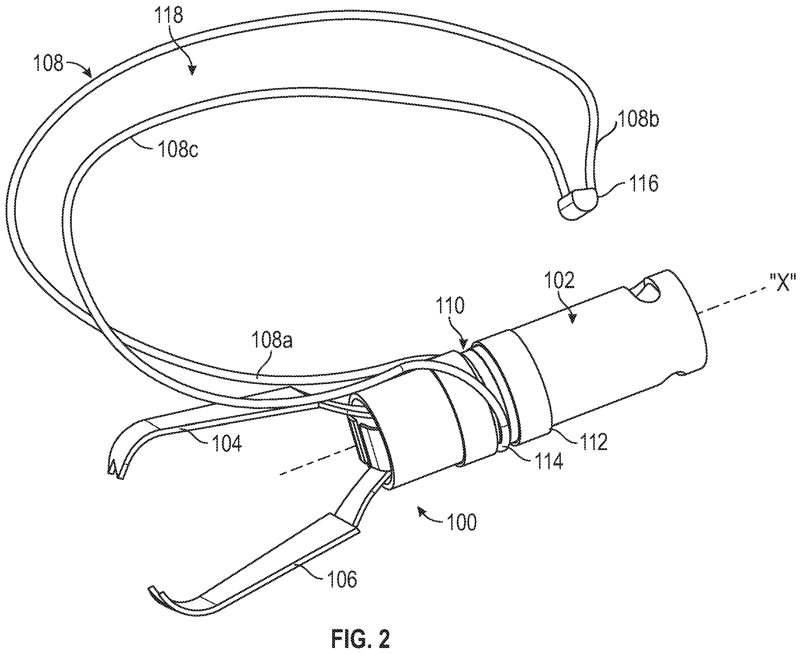

FIG. 1 illustrates a hand-held surgical instrument 10 for deploying a tissue clip 100 (FIG. 2). The surgical instrument 10 generally includes a handle 11, a shaft 12 extending distally from the handle 11, and a tissue clip 100 detachably coupled to a distal end portion 14 of the shaft 12. In some aspects, instead of the clip 100 being deployable from a hand-held instrument, the clip 100 may be deployed from a surgical robotic arm.

The surgical instrument 10 includes an actuation mechanism, such as, for example, a puller (not shown) axially movable within the shaft 12. The puller may have a proximal end operably coupled to a trigger 16 of the handle 11, such that an actuation of the trigger 16 proximally translates the puller. The puller may have a distal end detachably coupled to the tissue clip 100, such that proximal translation of the puller moves the tissue clip 100 from an open configuration to a closed configuration, as will be described. It is contemplated that the surgical instrument 10 may include any suitable actuation mechanism for deploying the tissue clip 100.

With reference to FIG. 2, the tissue clip 100 has a proximal body portion 102, such as, for example, a tubular body, first and second jaw members 104, 106 received in the proximal body portion 102, and a resilient member 108 coupled to the proximal body portion 102. The proximal body portion 102 is configured to be detachably coupled to the distal end 14 of the shaft 12. In aspects, the shaft 12 may have a release latch (not shown) coupled to the proximal body portion 102 and an actuation mechanism (e.g., a pull rod, not shown) for actuating the release latch to deploy the tissue clip 100 from the shaft 12.

The first and second jaw members 104, 106 are each coupled to the proximal body portion 102. Each of the first and second jaw members 104, 106 has a proximal end portion slidably received in a hollow interior of the proximal body portion 102, and a distal end portion disposed distally of the proximal body portion 102. The distal end portion of the jaw members 104, 106 may define teeth for assisting in grasping tissue between the jaw members 104, 106. The jaw members 104, 106 may be resiliently biased toward an open configuration by a biasing member (not shown). Alternately, the jaw members 104, 106 may be devoid of a resilient bias. The jaw members 104, 106 are axially movable relative to the proximal body portion 102 from a proximal position, in which the distal end portion of the jaw members 104, 106 are approximated toward one another, and a distal position, in which the distal end portion of the jaw members 104, 106 are spaced away from one another.

The resilient member 108 of the tissue clip 100 is fabricated from a shape memory material, such as, for example, copper-aluminium-nickel or nickel-titanium. In some aspects, the resilient member 108 may be fabricated from any suitable material configured to maintain a predefined shape. The resilient member 108 is fabricated from a wire that is folded over itself, with the folded end 114 formed into a loop to form a first end portion 108a of the resilient member 108, and the loose ends 116 of the wire being crimped together to form a second end portion 108b of the resilient member 108. The two folded portions of the wire may define a gap or aperture 118 therebetween. By spacing the two folded portions of the wire, an intermediate segment 108c of the resilient member 108 is more suitable for supporting tissue thereon. In some aspects, instead of fabricating the resilient member 108 from a folded wire, the resilient member 108 may be a thin, elongated sheet of resilient material. Other constructions of the resilient member 108 are also contemplated

The first end portion 108a of the resilient member 108 is rotationally supported on the proximal body portion 102. Specifically, the proximal body portion 102 defines an annular recess 110 having the first end portion 108a of the resilient member 108 slidably received therein. The first end portion 108a of the resilient member 108 is wrapped about the proximal body portion 102 and slidably received in the annular recess 110. As such, with the first end portion 108a of the resilient member 108 slidably received in the annular recess 110, the first end portion 108a is free to rotate about a longitudinal axis "X" defined by the proximal body portion 102 while being axially restrained to the proximal body portion 102.

The resilient member 108 assumes a looped configuration when in the deployed state. In particular, the first end portion 108a of the resilient member 108 extends distally from the annular recess 110 of the proximal body portion 102 and has a generally upwardly curved configuration. In aspects, the tissue clip 100 may have a collar 112 that clips onto the proximal body portion 102 and over the first end portion 108a of the resilient member 108 to hold the first end portion 108a in close relation with the proximal body portion 102. The intermediate segment 108c of the resilient member 108 extends from the first end portion 108a and curves laterally away from the longitudinal axis "X" of the proximal body portion 102 and in a generally proximal direction, such that in the deployed state, the intermediate segment 108c assumes an arcuate shape. The second end portion 108b of the resilient member 108 extends from the intermediate segment 108c and curves down toward the longitudinal axis "X" of the proximal body portion 102.

In use, with the tissue clip 100 coupled to the shaft 12 and the jaw members 104, 106 in the open configuration, the tissue clip 100 is positioned adjacent tissue (e.g., a lesion). The tissue is positioned between the jaw members 104, 106, whereupon the puller of the surgical instrument 10 retracts the jaw members 104, 106 proximally through the proximal body portion 102. An inner wall of the proximal body portion 102 acts on the jaw members 104, 106 to move the jaw members 104, 106 toward the closed configuration about the tissue. With the tissue grasped between the jaw members 104, 106, the puller is further retracted to detach the puller from the jaw members 104, 106, thereby releasing the proximal body portion 102 of the tissue clip 100 from the shaft 12 and leaving the tissue clip 100 at the surgical site. Other mechanisms for releasing the tissue clip 100 from the shaft 12 are also contemplated.

Upon releasing tissue clip 100 from the shaft 12, the resilient member 108 of the tissue clip 100 is allowed to transition from a stored state, in which the resilient member 108 is constrained within the shaft 12, to a deployed state. In the stored state, the resilient member 108 assumes a generally linear shape, and in the deployed state, the resilient member 108 moves toward its predefined, arcuate shape, in which the resilient member 108 bows outwardly from the proximal body portion 102.

After, during, or prior to grasping the tissue with the jaw members 104, 106, the resilient member 108 is positioned between the tissue and a muscle layer of a gastric wall, whereby the outward resilient bias of the resilient member 108 separates the grasped tissue from the gastric wall. To change an orientation of the resilient member 108 to better position the resilient member 108 between the grasped tissue and the gastric wall, the first end portion 108a of the resilient member 108 may be rotated about the proximal body portion 102. During rotation of the first end portion 108a, the intermediate segment 108c and the second end portion 108b is moved to a different side of the proximal body portion 102 until the desired orientation is achieved. With the tissue being maintained in spaced relation from the muscle layer by the resilient member 108, the grasped tissue may be dissected from the muscle layer.

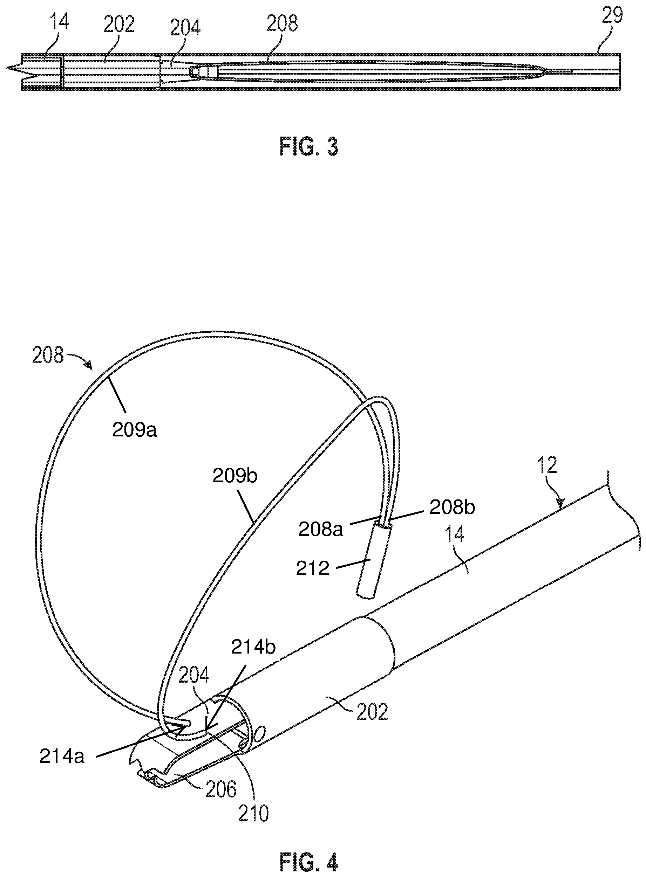

FIGS. 3 and 4 illustrate another embodiment of a tissue clip 200 deployable from the surgical instrument 10. The tissue clip 200 is similar to tissue clip 100 and will only be described in detail to elucidate differences between the two clips. The tissue clip 200 includes a proximal body portion, such as, for example, a tubular body 202, first and second jaw member 204, 206 received in the proximal body portion 202, and a resilient member 208. The proximal body portion 202 is configured to be detachably coupled to the distal end 14 of the shaft 12. As shown in FIG. 3, the surgical instrument 10 may have a sheath 20 detachably coupled to the shaft 14 and configured to encapsulate the resilient member 208 to selectively maintain the resilient member 208 in a stored, linear state. The tissue clip 200 differs from the tissue clip 100 by having the resilient member 208 directly attached to the jaw member 204. In aspects, the resilient member 208 may be formed from a folded wire, or in some aspects two discreet wires, having the folded end 210 non-rotatably fixed to the jaw member 204.

In aspects, the resilient member 208 consists of a single wire having two ends 208a, 208b that are attached to one another with a crimp 212. The jaw member 204 has a pair of holes 214a, 214b extending through a thickness thereof. Each of the holes 214a, 214b are spaced from one another a selected distance extending along a width of the jaw member 204. It is contemplated that the distance between the holes 214a, 214b in the jaw member 204 are selected so that two sections 209a, 209b of the single wire 208 are spaced from one another by a greater distance than the diameter of the tubular body 202 when deployed. In this way, upon the resilient member 208 engaging tissue, the resilient member 208 balances the tissue clip 200, thereby preventing the tissue clip 200 from rotating out of engagement with the tissue.

During manufacturing and/or assembly of the tissue clip 200, the two ends 208a, 208b of the single wire 208 are each respectively passed upwardly through the corresponding holes 214a, 214b in the jaw member 204 to establish a friction-fit engagement between the folded end 210 of the wire 208 and the jaw member 204. After passing the single wire/resilient member 208 through the holes 214a, 214b of the jaw member 204, the two ends 208a, 208b of the wire 208 are crimped together, thereby giving the resilient member 208 an enclosed loop shape. Attaching the resilient member 208 to the jaw member 204 via the holes 214a, 214b is a relatively cheap, easy, and effective means of coupling the resilient member 208 to the remainder of the tissue clip 200.

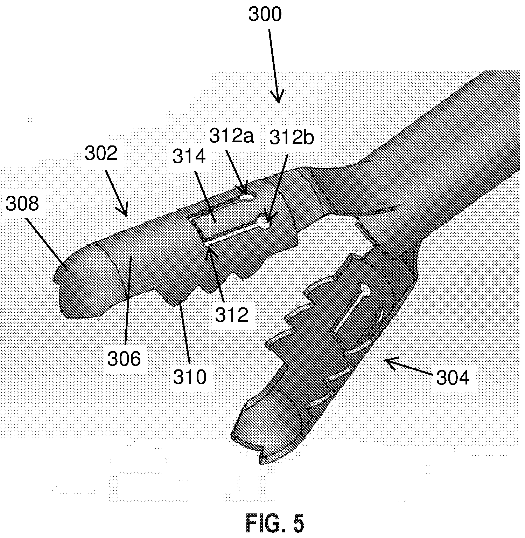

With reference to FIG. 5, another embodiment of a tissue clip 300 is illustrated. The tissue clip 300 is configured to be deployed from the surgical instrument 10 (FIG. 1) and is similar to tissue clip 200. The tissue clip 300 includes a pair of jaw members 302, 304 and a resilient member or wire 208 (FIG. 4) configured to be fixed to one of the jaw members 302, 304. Each of the jaw members 302, 304 has a body member 306 having a generally semi-hemispherical shape along a length thereof and a distal tip 308 that curves inwardly toward the other. Each of the jaw members 302, 304 has serrations 310 along the entire outer peripheral edge of the jaw members 302, 304 to assist with grasping tissue. The body member 306 of at least one of the jaw members 302 has a cutout 312 that forms a tab 314 and a pair of holes 312a, 312b for receipt of the resilient member 208 (FIG. 4).

During manufacturing and/or assembly of the surgical tissue clip 300, the tab 314 is bent outwardly and the folded end 210 of the resilient wire 208 is passed over the tab 314 and inserted into the holes 312a, 312b. The tab 314 is then folded down to capture the folded end 210 of the resilient member 208 in the holes 312a, 312b. In this way, the opposing ends 208a, 208b (FIG. 4) of the resilient member 208 may be crimped before attaching the resilient member 208 to the jaw member 302.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques).

## Claims

1. A tissue clip (100), comprising:
a proximal body portion (102);
first and second jaw members (104, 106) coupled to the proximal body portion and configured to move between an open configuration and a closed configuration to grasp tissue therebetween; and
a resilient member (108) coupled to the proximal body portion, the resilient member including a segment (108c) that is laterally spaced from the proximal body portion when the resilient member is in a deployed state;
**characterised in that** the resilient member includes:
a first end portion (108a) coupled to the proximal body portion and extending distally therefrom, the segment curving proximally and laterally from the first end portion; and
a second end portion (108b) extending from the segment.

2. The tissue clip according to claim 1, wherein the resilient member is transitionable between a stored state and the deployed state.

3. The tissue clip according to claim 2, wherein the segment of the resilient member assumes a linear shape when the resilient member is in the stored state, and the segment of the resilient member assumes an arcuate shape when the resilient member is in the deployed state.

4. The tissue clip according to claim 2, wherein the segment of the resilient member bows outwardly from the proximal body portion when the resilient member is in the deployed state.

5. The tissue clip according to claim 4, wherein the second end portion curves toward the proximal body portion.

6. The tissue clip according to claim 5, wherein the second end portion is disposed proximally of the first end portion.

7. The tissue clip according to claim 1, wherein the resilient member is configured to assume a looped configuration when in the deployed state.

8. The tissue clip according to claim 1, wherein the resilient member includes a wire having a predefined shape.

9. The tissue clip according to claim 1, wherein the resilient member has a first end portion rotationally coupled to the proximal body portion, and a second end portion, such that the resilient member is rotatable between a first position, in which the second end portion is disposed on a first side of the proximal body portion, and at least one second position, in which the second end portion is disposed on a second side of the proximal body portion.

10. The tissue clip according to claim 9, wherein the proximal body portion defines an annular recess having the first end portion of the resilient member slidably received therein.

11. The tissue clip according to claim 1, wherein the resilient member includes first and second wires defining a gap therebetween.

12. The tissue clip according to claim 11, wherein the gap is greater than a diameter of the proximal body portion.

## Patentansprüche

1. Gewebeklammer (100), umfassend:
einen proximalen Körperabschnitt (102);
erste und zweite Backenelemente (104, 106), die mit dem proximalen Körperabschnitt gekoppelt und konfiguriert sind, um sich zwischen einer offenen und einer geschlossenen Konfiguration zu bewegen, um dazwischen Gewebe zu greifen; und
ein elastisches Element (108), das mit dem proximalen Körperabschnitt gekoppelt ist, wobei das elastische Element ein Segment (108c) einschließt, das lateral vom proximalen Körperabschnitt beabstandet ist, wenn sich das elastische Element in einem angewendeten Zustand befindet;
**dadurch gekennzeichnet, dass** das elastische Element einschließt:
einen ersten Endabschnitt (108a), der mit dem proximalen Körperabschnitt gekoppelt ist und sich distal davon erstreckt, wobei das Segment proximal und lateral vom ersten Endabschnitt aus gekrümmt ist; und
einen zweiten Endabschnitt (108b), der sich vom Segment aus erstreckt.

2. Gewebeklammer nach Anspruch 1, wobei das elastische Element zwischen einem gelagerten Zustand und einem angewendeten Zustand überführbar ist.

3. Gewebeklammer nach Anspruch 2, wobei das Segment des elastischen Elements eine lineare Form annimmt, wenn sich das elastische Element im gelagerten Zustand befindet, und das Segment des elastischen Elements eine bogenförmige Form annimmt, wenn sich das elastische Element im angewendeten Zustand befindet.

4. Gewebeklammer nach Anspruch 2, wobei sich das Segment des elastischen Elements vom proximalen Körperabschnitt nach außen wölbt, wenn sich das elastische Element im angewendeten Zustand befindet.

5. Gewebeklammer nach Anspruch 4, wobei der zweite Endabschnitt in Richtung des proximalen Körperabschnitts gekrümmt ist.

6. Gewebeklammer nach Anspruch 5, wobei der zweite Endabschnitt proximal zum ersten Endabschnitt angeordnet ist.

7. Gewebeklammer nach Anspruch 1, wobei das elastische Element konfiguriert ist, um im angewendeten Zustand eine Schleifenkonfiguration anzunehmen.

8. Gewebeklammer nach Anspruch 1, wobei das elastische Element einen Draht mit einer vordefinierten Form einschließt.

9. Gewebeklammer nach Anspruch 1, wobei das elastische Element einen ersten Endabschnitt aufweist, der drehbar mit dem proximalen Körperabschnitt gekoppelt ist, und einen zweiten Endabschnitt, so dass das elastische Element zwischen einer ersten Position, in der der zweite Endabschnitt auf einer ersten Seite des proximalen Körperabschnitts angeordnet ist, und mindestens einer zweiten Position, in der der zweite Endabschnitt auf einer zweiten Seite des proximalen Körperabschnitts angeordnet ist, drehbar ist.

10. Gewebeklammer nach Anspruch 9, wobei der proximale Körperabschnitt eine ringförmige Aussparung definiert, in der der erste Endabschnitt des elastischen Elements verschiebbar aufgenommen ist.

11. Gewebeklammer nach Anspruch 1, wobei das elastische Element erste und zweite Drähte einschließt, die zwischen sich einen Spalt definieren.

12. Gewebeklammer nach Anspruch 11, wobei der Spalt größer ist als ein Durchmesser des proximalen Körperabschnitts.

## Revendications

1. Agrafe de tissu (100), comprenant :
une partie de corps proximale (102) ;
un premier et un second élément de mâchoire (104, 106) accouplés à la partie de corps proximale et conçus pour se déplacer entre une configuration ouverte et une configuration fermée afin de saisir le tissu entre eux ; et
un élément élastique (108) accouplé à la partie de corps proximale, l'élément élastique comportant un segment (108c) qui est latéralement espacé de la partie de corps proximale lorsque l'élément élastique est dans un état déployé ;
**caractérisée en ce que** l'élément élastique comporte :
une première partie d'extrémité (108a) accouplée à la partie de corps proximale et s'étendant distalement à partir de celle-ci, le segment s'incurvant proximalement et latéralement à partir de la première partie d'extrémité ; et
une seconde partie d'extrémité (108b) s'étendant à partir du segment.

2. Agrafe de tissu selon la revendication 1, dans laquelle l'élément élastique peut alterner entre un état stocké et l'état déployé.

3. Agrafe de tissu selon la revendication 2, dans laquelle le segment de l'élément élastique prend une forme linéaire lorsque l'élément élastique est à l'état stocké, et le segment de l'élément élastique prend une forme arquée lorsque l'élément élastique est à l'état déployé.

4. Agrafe de tissu selon la revendication 2, dans laquelle le segment de l'élément élastique s'incline vers l'extérieur à partir de la partie de corps proximale lorsque l'élément élastique est à l'état déployé.

5. Agrafe de tissu selon la revendication 4, dans laquelle la seconde partie d'extrémité s'incurve vers la partie de corps proximale.

6. Agrafe de tissu selon la revendication 5, dans laquelle la seconde partie d'extrémité est disposée de manière proximale par rapport à la première partie d'extrémité.

7. Agrafe de tissu selon la revendication 1, dans laquelle l'élément élastique est conçu pour prendre une configuration en boucle lorsqu'il est à l'état déployé.

8. Agrafe de tissu selon la revendication 1, dans laquelle l'élément élastique comporte un fil ayant une forme prédéfinie.

9. Agrafe de tissu selon la revendication 1, dans laquelle l'élément élastique a une première partie d'extrémité accouplée en rotation à la partie de corps proximale, et une seconde partie d'extrémité, de telle sorte que l'élément élastique peut tourner entre une première position, dans laquelle la seconde partie d'extrémité est disposée sur un premier côté de la partie de corps proximale, et au moins une seconde position, dans laquelle la seconde partie d'extrémité est disposée sur un second côté de la partie de corps proximale.

10. Agrafe de tissu selon la revendication 9, dans laquelle la partie de corps proximale définit un renfoncement annulaire ayant la première partie d'extrémité de l'élément élastique reçue de manière coulissante à l'intérieur de celui-ci .

11. Agrafe de tissu selon la revendication 1, dans laquelle l'élément élastique comporte un premier et un second fils définissant un espace entre eux.

12. Agrafe de tissu selon la revendication 11, dans laquelle l'espace est supérieur à un diamètre de la partie de corps proximale.
